# EUROPEAN PATENT APPLICATION

(11) **EP 2 228 411 A1**
(43) Date of publication of application: **15.09.2010**
(21) Application number: 09002501.6
(22) Date of filing: 21.02.2009
(51) Int. Cl.: C09B 61/00, C09B 67/22, C09B 67/02, A23L 1/275, A23L 2/58, A61K 8/30

(54) **Food grade colouring agent**

(71) Applicant: Rugeris, Jess Edward, West Molesey Surrey KT8 1QU (GB)
(72) Inventor: Rugeris, Jess Edward, West Molesey Surrey KT8 1QU (GB)
(74) Representative: Schaad, Balass, Menzl & Partner AG

(57) **Abstract**

The present invention relates to a method for the preparation of a food grade colouring agent by drying one or more coloured vegetable materials selected from the group consisting of red cabbage, beetroot, lemon, black currant, red currant, strawberry, blackberry, blueberry, and saffron under conditions resulting in a predetermined colour. The food grade colouring agent thus obtained can be used for the preparation of coloured food, drinks, pharmaceutical, and cosmetic products.

## Description

The present invention relates to a food grade colouring agent, to a method for the preparation thereof, and to its use for the preparation of coloured goods.

Nowadays, a wide range of food grade colouring agents is used by the food industry in order to enhance the optical appearance of food.

Of the known food grade colouring agents, only a few are natural, such as betanoin or chlorophyll, for instance. Most food grade colouring agents are either synthetic analogs of naturally occurring substances - so-called nature identical substances - or are fully artificial.

The use of some of the artificial and synthetic colouring agents, e.g. of the azo dyes, for colouring food is highly controversial, as they are known or suspected to be allergenic, carcinogenic, or to have other undesired side effects on the human body or the environment.

It is therefore a problem of the present invention to provide food grade colouring agents in a wide range of colours, which are absolutely safe to use.

The problem is solved by the method according to claim 1, the food grade colouring agent according to claim 7 and the use of the colouring agent according to claim 12. Preferred embodiments are subject of the dependent claims.

The present invention provides a method for the preparation of a food grade colouring agent. The food grade colouring agent is obtained by drying one or more coloured vegetable materials under conditions resulting in a predetermined colour. The one or more coloured vegetable materials are selected from the group consisting of red cabbage, beetroot, lemon, black currant, red currant, strawberry, blackberry, blueberry, cranberry, and saffron.

The method of the present invention affords a potent colouring agent, which is derived from coloured vegetable materials. It is therefore absolutely safe for use in food, drinks, and other products, which are applied to the human body, such as pharmaceutical or cosmetic products. In addition, the method of the present invention allows for a very straight forward, cost effective preparation of the colouring agent.

The coloured vegetable materials are dried under conditions resulting in a predetermined colour. By drying the vegetable materials, it is possible to prepare a very concentrated colouring agent, thus minimizing the amount of colouring agent necessary to colour a certain amount of a food product. The colour of the colouring agent of the present invention is especially stable and insensitive towards oxidation, as it is still bound by the dried vegetable material. At the same time, the method of the present invention allows for the preparation of a food grade colouring agent having a predetermined colour by adjustment of the drying conditions, such as temperature, drying method or drying time. A person skilled in the art is able to determine the exact conditions using pertinent preliminary tests.

The term "vegetable materials", as used throughout this application, includes not only entire fruits or vegetables, but also selected parts of fruits or vegetables, such as leafs or petals. The vegetable materials, which are preferably used in the method of the present invention, are particularly colourful and afford a potent colouring agent when dried. In addition, it is possible to prepare a wide range of different colours starting from one or more of the above coloured vegetable materials.

In order to obtain a particularly safe colouring agent, it is possible to use organic vegetable materials.

In a preferred embodiment, the coloured vegetable materials are air or freeze dried. Alternatively, the coloured vegetable materials can also be spray dried. These drying methods allow for a fast and gentle drying of the vegetable materials, affording the colouring agent in the desired colour and with a high colouring strength.

Air drying is particularly favourable for drying the coloured vegetable materials, as it allows preserving the desired colours while most of the aroma is removed. In addition, the colouring agent prepared by air drying is particularly stable. Even after several months of storage under air, no oxidation is observed. Freeze drying, on the other hand, allows preserving some of the flavours originally contained in the coloured vegetable materials. It is therefore possible to obtain a colouring agent from lemon zest having a slight lemon smell. Preferably, freeze dried colouring agents are stored under vacuum to prevent oxidation.

In order to facilitate the drying process, it is preferred that the coloured vegetable materials are cut or ground before being dried. In this manner, the surface area of the vegetable materials is enhanced and the drying process accelerated. The method of this preferred embodiment affords the colouring agent in relatively small particles having a large surface area, which can easily be measured and dosed. Furthermore, the enlarged surface area also improves the colouring agent's colouring properties, since the colour is released through the surface.

Alternatively, it is also possible that the coloured vegetable materials are cut or ground after the drying.

In a preferred embodiment, two or more coloured vegetable materials are mixed after having been dried. By mixing several vegetable materials, it is possible to prepare food grade colouring agents in a large variety of different colours and in various shades. Thus, the colour can be adjusted to the needs of the industry and the desires of the consumers.

Alternatively, it is also possible that two or more coloured vegetable materials are mixed prior to drying.

In a preferred embodiment of the present invention, the dried vegetable material is inserted into a gas and/or liquid permeable package, preferably a sachet or pod. Inserting the dried vegetable material into such a package offers a wider range of advantages: The colouring agent can be allotted in the desired amounts into each package to provide a single dose. Several single dose packages can then be further packaged into a container suitable for storage, transport and sale of the colouring agent. When using the colouring agent, it is not necessary to touch the dried vegetable substances directly, but only the surrounding package, thus avoiding contamination. Furthermore, during the actual colouring process, the gas and/or liquid permeable package containing the food grade colouring agent can be inserted into a liquid or gas, which is able to extract the colour. The colour itself is "washed out" of the package by the liquid or gas, whereas the solid residues of the colouring agent are kept inside the package and can easily be removed from the extraction liquid or gas.

In a further aspect, the present invention also relates to a food grade colouring agent obtained by the method of the present invention. This colouring agent is absolutely safe for used in food, drinks, and other everyday life products, which are applied to the human body, such as pharmaceutical or cosmetic products, for instance.

In a preferred embodiment, at least the colour of the colouring agent is soluble in hot water and/or steam. This allows for extraction of the colour using hot water and/or steam immediately before the introduction into the product to be coloured. Preferably, only the colour is soluble in hot water and/or steam, whereas the rest of the colouring agent is not soluble and can easily be removed from the water or steam by filtration after use.

In a preferred embodiment, the food grade colouring agent of the present invention has no taste. In general, a tasteless substance will also be odourless. In the food industry and, in particular, in the beverage industry, the use of colouring agents is common practice. In order to conserve the original smell and taste of the food or drink, however, it is important that the colouring agent itself does not have a taste.

In a preferred embodiment, the food grade colouring agent according to the present invention comprises at least dried red cabbage. Colouring agents comprising dried red cabbage, optionally in combination with one or more other coloured vegetable materials, offer a wide range of colours: Depending on what other coloured vegetable materials are added, various shades of blue, turquoise, green, orange, red, and pink can be obtained. From a colouring agent, which comprises at least dried red cabbage, the colour can easily be extracted using hot water or steam. In addition, dried red cabbage does not have a taste and is therefore particularly well suited for the preparation of coloured drinks or ice-cubes. A further advantage of a food grade colouring agent according to the present invention, which comprises dried red cabbage, is that it is non-staining: If a liquid coloured with the colouring agent is poured through a white cotton cloth, for instance, the cloth is not stained and remains white.

In a further preferred embodiment, the food grade colouring agent of the present invention comprises at least dried lemon, in particular from lemon zest or lemon juice. A colouring agent prepared from lemon zest affords an intense yellow colour. In combination with other coloured vegetable materials, dried lemon zest can be used to produce mixed colours such as orange or green. Lemon juice, on the other hand, will provide a pink colour when added to dried red cabbage leaves or parts thereof. Food grade colouring agents comprising dried lemon possess a slight lemon smell and taste, which may be particularly interesting if the colouring agent is used for the preparation of coloured drinks or ice-cubes.

In a further aspect, the present invention also relates to the use of the food grade colouring agent for the preparation of a coloured liquid by extracting the colouring agent with hot water or steam. Preferably, the water used for extraction has a temperature of at least 45 °C, more preferably of at least 70 °C. By extracting the colouring agent of the present invention with hot water or steam, the colour is dissolved in the water or steam and - in the case of steam, after condensation - a coloured liquid is obtained. In addition, at a water temperature of more than 70 °C, pathogens potentially contained in the water are killed. Alternatively, it is also possible to use the food grade colouring agent of the present invention for colouring a non-water-based liquid.

The coloured liquid obtained by extraction of the food grade colouring agent of the present invention is preferably used for the preparation of coloured ice-cubes, coloured drinks or coloured food. Alternatively, it can also be used to prepare coloured pharmaceutical or cosmetic products. In all these cases, the characteristics of the colouring agent of the present invention described above - namely simple and cost effective preparation, availability of a wide range of colours in different shades, and complete safety for human and animal beings - are particularly advantageous.

### Examples

### Drying apparatus

The coloured vegetable materials were in general air dried, using a 28x28x76 cm cardboard box with a drying rack in the closed area, an internal 60 W light bulb, and breathing vents on top. The coloured vegetable material was placed on the drying rack and the light bulb was used to create dry heat with a temperature of about 35 °C.

### Example 1: Preparation of a blue food grade colouring agent

500 g of fresh, raw red cabbage leaves were air dried using the drying apparatus described above at a drying temperature of 35 °C over a period of 4 days to afford 50 g of dried red cabbage. The dried red cabbage was granulated and, in an amount of 1 g each, inserted into sachets. One sachet was used to produce up to 9 1 of deep blue water by extraction with hot water having a temperature of 70 to 100 °C. The blue water was used for the preparation of blue ice-cubes.

### Example 2: Preparation of a green food grade colouring agent

1 g of dried and granulated red cabbage, which was obtained by the method described in example 1, was mixed in a ratio of 5:1 with dried saffron. The mixture was dipped into hot water having a temperature of 70 °C for 1 second and then allowed to air dry at a temperature of 35 °C over a period of 24 hours. The dried mixture was inserted into a sachet, which was used to produce up to 9 1 of lime green water by extraction with hot water having a temperature of 70 to 100 °C. The lime green water was used for the preparation of lime green ice-cubes.

### Example 3: Preparation of a pink food grade colouring agent

1 g of dried red cabbage, which was obtained by the method described in example 1, was soaked with 5 ml of lemon juice and was then allowed to air dry at a temperature of 35 °C over a period of 24 hours. The dried mixture was granulated and, in an amount of 1 g each, inserted into sachets. One sachet was used to produce up to 9 1 of pink water by extraction with hot water having a temperature of 70 to 100 °C. The pink water was used for the preparation of pink ice-cubes.

### Example 4: Preparation of a purple food grade colouring agent

100 g of cooked or raw beetroot was air dried using the drying apparatus described above at a drying temperature of 35 °C over a period of 4 days to afford 15 g of dried beetroot. The dried beetroot was granulated.

0.5 g of dried and granulated red cabbage, which was obtained by the method described in example 1, was mixed with 0.5 g of dried and granulated beetroot. The mixture was inserted into a sachet, which was used to produce up to 9 1 of purple water by extraction with hot water having a temperature of 70 to 100 °C. The purple water was used for the preparation of purple ice-cubes.

In this context, the use of cooked beetroot proved advantageous, as the derived purple ice-cubes were less tainting upon melting.

### Example 5: Preparation of an orange food grade colouring agent

1 g of dried red cabbage, which was obtained by the method described in example 1, was mixed in a ratio of 5:1 with dried saffron. 4 to 5 drops of lemon juice were added to the mixture and it was then allowed to air dry. The dried mixture was granulated and, in an amount of 1 g each, inserted into sachets. Each sachet can be used to produce up to 7 1 of orange or gold coloured water by extraction with hot water having a temperature of 70 to 100 °C. The orange water was used for the preparation of orange ice-cubes.

### Example 6: Preparation of a turquoise food grade colouring agent

1 g of dried red cabbage, which was obtained by the method described in example 1, was mixed in a ratio of 5:1 with dried saffron. The mixture was granulated and inserted into a sachet, which can be used to produce about 1.5 to 2 1 of turquoise water by extraction with hot water having a temperature of 70 to 100 °C. The turquoise water was used for the preparation of turquoise ice-cubes.

### Example 7: Preparation of a yellow food grade colouring agent having a lemon smell

30 g of lemon zest, which was obtained from mature lemons having a dark yellow colour, was air dried using the drying apparatus described above at a temperature of 35 °C over a period of 24 hours to afford 3 g of dried lemon zest. The dried lemon zest was granulated and, in an amount of 1 g each, inserted into sachets. One sachet was used to produce about 1.5 to 2 1 of yellow water having a lemon smell. The yellow water was used for the preparation of yellow ice-cubes.

### Further Colours

In examples 1, 2, and 6, the preparation of a blue, green, and turquoise food grade colouring agent has been described, respectively. By altering the ratio of red cabbage to saffron, as will be well understood by a person skilled in the art, various shades of green, turquoise, and blue colouring agents can be obtained.

In examples 2, 5, and 6, the preparation of a green, orange, and turquoise food grade colouring agent has been described, respectively. By replacing saffron with dried lemon zest, food grade colouring agents of similar colours but with a lemon smell can be obtained.

## Claims

1. Method for the preparation of a food grade colouring agent by drying one or more coloured vegetable materials selected from the group consisting of red cabbage, beetroot, lemon, black currant, red currant, strawberry, blackberry, blueberry, cranberry, and saffron under conditions resulting in a predetermined colour.

2. Method for the preparation of a food grade colouring agent according to claim 1, whereby the one or more coloured vegetable materials are air or freeze dried.

3. Method for the preparation of a food grade colouring agent according to one of the preceding claims, whereby the one or more coloured vegetable materials are cut or ground before being dried.

4. Method for the preparation of a food grade colouring agent according to one of the preceding claims, whereby two or more coloured vegetable materials are mixed after having been dried.

5. Method for the preparation of a food grade colouring agent according to one of the preceding claims, whereby the dried vegetable material is inserted into a gas and/or liquid permeable package.

6. Method for the preparation of a food grade colouring agent according to claim 5, whereby the gas and/or liquid permeable package is a sachet or pod.

7. Food grade colouring agent obtained by the method of one of the preceding claims.

8. Food grade colouring agent according to claim 7, whereby at least the colour of the colouring agent is soluble in hot water and/or steam.

9. Food grade colouring agent according to claim 7 or 8, whereby it has no taste.

10. Food grade colouring agent according to one of claims 7 to 9, whereby it comprises at least dried red cabbage.

11. Food grade colouring agent according to one of claims 7 to 10, whereby it comprises at least dried lemon.

12. Use of the food grade colouring agent according to one of claims 7 to 11 for the preparation of a coloured liquid by extracting the colouring agent with hot water or steam.

13. Use of the food grade colouring agent according to claim 12, whereby the coloured liquid is used for the preparation of coloured ice-cubes.

14. Use of the food grade colouring agent according to claim 12, whereby the coloured liquid is used for the preparation of coloured drinks.

15. Use of the food grade colouring agent according to claim 12, whereby the coloured liquid is used for the preparation of coloured food.

16. Use of the food grade colouring agent according to claim 12, whereby the coloured liquid is used for the preparation of a coloured pharmaceutical product.
